# EUROPEAN PATENT APPLICATION

(11) **EP 2 284 280 A1**
(43) Date of publication of application: **16.02.2011**
(21) Application number: 08805380.6
(22) Date of filing: 31.07.2008
(51) Int. Cl.: C12Q 1/68

(54) **PRIMERS, METHOD AND SET OF TOOLS FOR DETERMINING THE FUNCTIONALITY OF THE HUMAN THYMUS**

(30) Priority: 02.08.2007 ES 200702224
(71) Applicant: Fundacion Reina Mercedes Para La Investigacion Sanitaria, 41013 Sevilla (ES); Fundación para la Investigación y Prevención del SIDA en España, 28080 Madrid (ES)
(72) Inventor: LEAL NOVAL, Manuel, E-41013 Sevilla (ES); MUÑOZ FRANCO, Jaime, E-41013 Sevilla (ES); FERRANDO MARTÍNEZ, Sara, E-41013 Sevilla (ES)
(74) Representative: Pons Ariño, Angel
(86) International application number: PCT/ES2008/000559
(87) International publication number: WO 2009/027568

(57) **Abstract**

Determining thymic function in adults has attracted increasing interest in the context of diseases that affect the immunological system and, in particular, diseases that produce lymphopenias. The invention relates to a set of primers for determining the functionality of the human thymus by calculating the quotient between two types of TREC content, as well as to a method based on multiplex nested PCR which uses the aforementioned primers and can be used to determine the TREC quotient measured in samples of human blood or tissue. The invention is advantageous over methods known in the prior art in terms of reducing experimental errors and the time and costs involved.

## Description

### OBJECT OF THE INVENTION

The determination of thymic function in adults has attracted increasing interest in the context of immunological system diseases, and in particular in those diseases that cause lymphopenias. The present invention is advantageous over methods known in the prior art in terms of reducing experimental errors and the time and savings involved.

A first object of the present invention is a set of primers for determining the functionality of the human thymus by calculating the quotient between the delta and beta-type TREC content (T-cell rearrangement excision circle).

A second object of the present invention is a method based on multiplex nested PCR which uses said primers and can be used to determine the TREC quotient measured in samples of human blood or tissues, particularly in the thymus and in peripheral blood, a parameter which is an indicator of the functionality of the thymus due to its direct correlation with the percentage of double positive thymocytes.

The last object of the present invention is set of tools to implement said method based on multiplex nested PCR.

### STATE OF THE ART

During T-lymphocyte generation (TL), the alpha and beta chains that for the TL clonotypical receptor (T-Cell Receptor, or TCR), are generated by the random recombination of gene segments, type V, D and J.

During this recombination, the DNA that separates it is eliminated from the chromosome in the shape of a circle, forming said TREC (T-cell rearrangement excision circle). These TREC are DNA fragments that exist independently from the chromosome, which means they lack replication origins. When the cell carrying it proliferates, the TREC does not replicate and is conserved by only one of the cells generated. Since the only way of generating TREC is by using the gene reordering that occurs in the thymus, they have been considered as molecular markers of thymic function.

There are hundreds of different TREC, as many as there are possibilities of compatible recombination between the gene segments V, D and J of the TCR chains. From all of them, one is particularly well known which, due to its formation characteristics, is extremely frequent between the TL that abandon the thymus, which is the δRec-ψJά TREC, or more commonly signal-joint TREC or delta TREC (Douek et al. "Changes in thymic function with age and during the treatment of HIV infection"; Nature 1998, Vol. 396, 690-695). Traditionally, delta TREC have been used as markers of thymic function, finding numerous publications that use them, most of them in the context of HIV infection or in bone marrow transplant in immunodepressed patients.

However, many of these publications indicate a limitation of the technique for measuring thymic function, in particular in the case of the HIV infection [Hazemberg et al. "Increased cell division but not thymic dysfunction rapidly affects the T-cell receptor excision circle content of the naive T cell population in HIV- 1 infection" 2000 Nature Medicine Vol. 6 (8) 1036- 1042]. Although the absolute number of TREC cannot increase independently from the thymus, the frequency of carrier cells in peripheral blood can change due to proliferation and/or apoptosis of the mature TL. These parameters are increased in HIV infection, and may decrease with the antiretroviral treatment. These changes may increase the frequency of carrier cells of TREC without thymic function having increased, simply due to reduction in activated TL, lacking TREC (diluting cells).

Recently, a variation has been published in the technique which overcomes this difficulty: it determines the quotient between two types of TREC: the traditionally measured delta and beta TREC (Dion et al. HIV Infection Rapidly Induces and Maintains a Substantial Suppression of Thymocyte Proliferation; 2004 Immunity, Vol. 21, 757-768), based on the following model: the generation in the thymus of a TL implies the step ordered by different phases of differentiation characterized by the sequential expression of surface markers that define the triple negative (TN₁ CD3" CD4" CD8"), double positive (DP, CD4⁺CD8⁺) and single positive (SP, CD3⁺CD4⁺ or CD3⁺CD8⁺) populations as well as by the following sequence: reordering of the beta chain, cell proliferation phase and reordering of the alpha chain. After the productive reordering of the beta chain during the TN phase all cells have beta TREC as by-product of said reordering, and then a phase of proliferation and transformation in DP cells commences.

This expansion makes it possible to increase the number of cells with productive reordering of the beta TCR, but they have as consequence the dilution in the number of beta TREC in said population of thymocytes. Next, and during the alpha chain reordering process, the delta TREC is generated, fixing at this point the delta/beta TREC quotient, a quotient which will characterize the cohort of TL which will later exit the thymus.

The hypothesis of Dion's article establishes that the degree of proliferation between the TN and DP stages is variable and that it can be estimated by analysing the quotient in the TL exported from the thymus. We can suppose that there is a direct relation between the degree of intrathymic proliferation and the total cells exported by the thymus (Almeida et al. T Cell Homeostasis: Thymus Regeneration and Peripheral T Cell Restoration in Mice with a Reduced Fraction of Competent Precursors. 2001. Journal of Experimental Medicine, Vol 194, 591-599) and that this quotient is not altered by peripheral expansion of the TL. This relation has been proven, establishing a direct correlation between the percentage of DP thymocytes as indicative of its functionality, and the TREC quotient measured in the thymus and/or in the peripheral blood.

The problem of this new approach is that, unlike the delta TREC, which is unique, there are up to thirteen different possibilities for recombination to generate beta TREC, which requires individually determining each possible TREC separately and adding the partial values. The technique proposed by Dion measures ten of the thirteen beta TREC, each one using an independent quantitative PCR. Next, they are all added together to obtain the total beta TREC of the sample. According to the originally described technique, to determine the quotient between the delta TREC and the sum of beta TREC 11 conventional PCRs and 55 real time PCR per samples were used (performing the measurements in triplicate). With the present approach by multiplex PCR, six of the thirteen beta TREC are co-amplified simultaneously and re-amplified together with the multiplex reaction in the same PCR tube. In this way, to determine the quotient of a sample (also in triplicate), only six conventional and three real time PCRs are needed.

### EXPLANATION OF THE INVENTION

A first object of the present invention is a set of primers for determining the functionality of the human thymus by calculating the quotient between the delta and beta TREC content; said set includes at least three primers each one of which have a sequence of nucleotides which is complementary with a region between nucleotides 187957 to 190719 of the sequence deposited in the Genbank database with access number U66061, as well as a sequence of nucleotides at their 5' end which is substantially non-complementary with any known region of the human genome. Preferably, the primers that form the set have at their 3' end a same common sequence to all of them which is either identical, or complementary, to one of sequences SEQ. ID. 01 to SEQ. ID. 99 (see tables 1 and 2) and more preferably the sequence common to all the primers at its 3' end is SEQ. ID. 07. Additionally, the set of primers includes at least one primer which contains one of sequences SEQ. ID. 100 to SEQ. ID. 112, and preferably simultaneously:
a) at least one primer which contains one of sequences SEQ. ID. 100 to SEQ. ID. 105.
b) at least one primer which contains one of sequences SEQ. ID. 106 to SEQ. ID. 107.
c) a primer which contains sequence SEQ. ID. 108.
d) at least one primer which contains one of sequences SEQ. ID. 109 to SEQ. ID. 112.

In a preferred embodiment of the invention, said set of primers is formed by:
a) six primers which contain sequences SEQ. ID. 100 to SEQ. ID.105.
b) two primers which contain sequences SEQ. ID. 106 and SEQ. ID. 107.
c) a primer which contains sequence SEQ. ID. 108.
d) four primers which contain sequences SEQ. ID. 109, SEQ. ID. 110, SEQ. ID. 111 and SEQ. ID. 112.

A second object of the present invention is a method for determining the functionality of the human thymus which includes simultaneous amplification stages of more than one beta or delta-type TREC by polymerase chain reaction (multiplex PCR). Said method comprises:
a) performing an amplification reaction from a cellular DNA preparation using a specific primer of a sequence common to the set of TREC of the beta family to be amplified, and a set of specific primers for each individual TREC of the beta family to be amplified, which have at its 5' end an identical sequence for all of them and which is substantially non-complementary with any known region of the human genome;
b) performing in parallel, but independently, an amplification reaction from a cellular DNA preparation using a pair of specific primers of the sequence of specific bases of a delta-type TREC;
c) performing a single amplification reaction from a mixture of aliquots of said previous amplification reactions, wherein at least one primer is used which comprises the sequence present at the 5' end of the set of primers used in the stage a). The aliquots of the amplification products of the first amplification reactions are used directly or diluted at least in the proportion 1 :2, preferably 1 :20.

The detection of the products of the polymerase chain reaction of the set of delta and beta TREC are performed in real time using a hybridization probe common for all beta TREC and a hybridization probe specific for delta TREC, the signals of both probes being distinguishable. Preferably, said hybridization probes for the detection of the PCR products of the set of delta and beta TREC, are fluorescence probes.

In a preferred embodiment, the primers used correspond to sequences SEQ. ID. 100 to SEQ. ID. 112 and the method is carried out according to the following stages:
a) a first amplification round of six β-TREC in a single multiplex PCR reaction using six sense primers (SEQ. ID.100 to SEQ. ID. 105) specific for each β-TREC and a single antisense primer (SEQ. ID. 106) specific for a constant region of six β-TREC and, in parallel, a first amplification round of δ-TREC using PCR using the primers which contain sequences SEQ. ID. 109 and SEQ. ID. 110, all from cellular DNA samples, particularly of blood or thymus cells;
b) a second amplification round set of aliquot dilutions of at least 1:2, preferably 1:20, of each one of the amplifications performed in the previous step using a single multiplex PCR which uses the primers which contain sequences SEQ. ID. 107 and SEQ. ID. 108 for the β-TREC amplified in the previous step and the primers which contain sequences SEQ. ID. 111 and SEQ. ID. 112 for the δ-TREC amplified in the previous step;
c) calculation of the quotient between the quantity of δ-TREC and the total quantity of β-TREC.

In parallel to the test samples a serialized dilution is amplified of a straight-line pattern which contains known concentrations of the products of the first δ-TREC or β-TREC amplification reaction, so that it can be compared with the values obtained with the test samples in the threshold amplification cycle of the second real time PCR, obtaining the concentrations from the interpolation of the threshold cycles of each test sample with the logarithmic-linear adjustment of the threshold cycle of the straight-line pattern. The absolute number of copies of δ-TREC or β-TREC per microlitre of cellular DNA preparation is normalized from the number of copies of the beta-globin gene, with two copies of said gene per cell, expressing the frequency of said TREC as number of circles per million total cells.

A third object of the present invention is a set of tools for determining the functionality of the human thymus using the method of the invention, which includes:
a) set of primers which contain sequences SEQ. ID. 100 to SEQ. ID. 112.
b) hybridization probes which make it possible to differentiate between the β-TREC and the δ-TREC, preferably fluorescence probes.
c) cloning vectors with known concentrations of β-TREC and δ-TREC which allow construction of a straight line pattern.

### BRIEF DESCRIPTION OF THE FIGURES

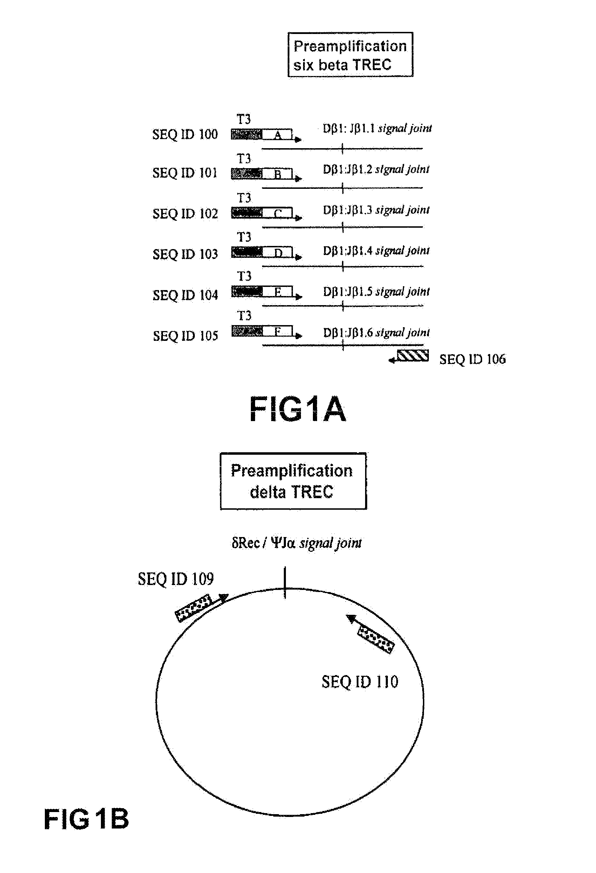

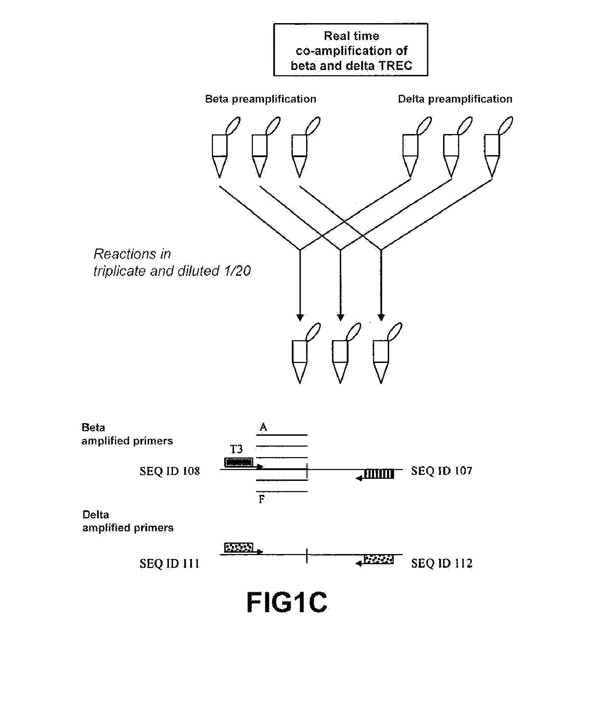

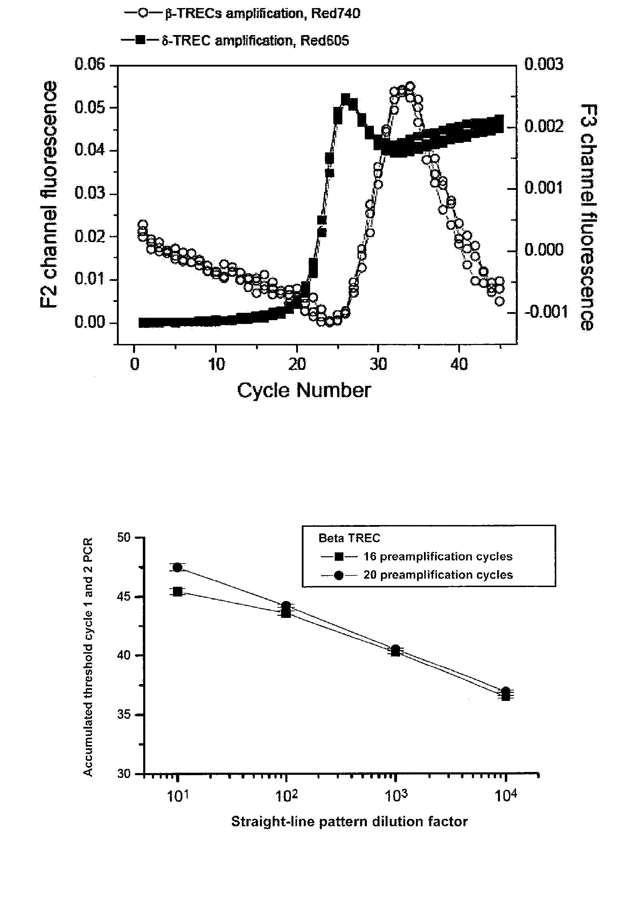

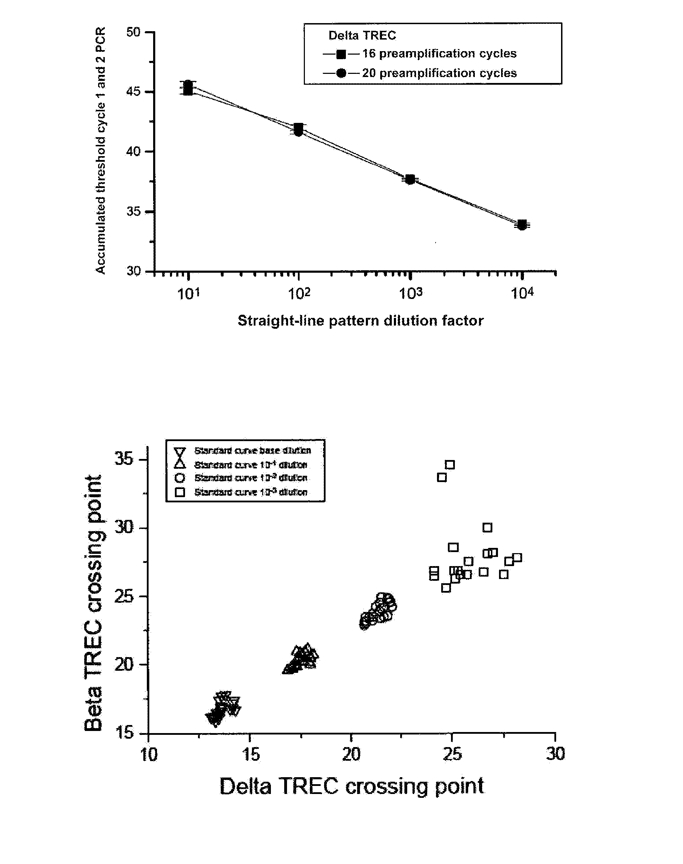

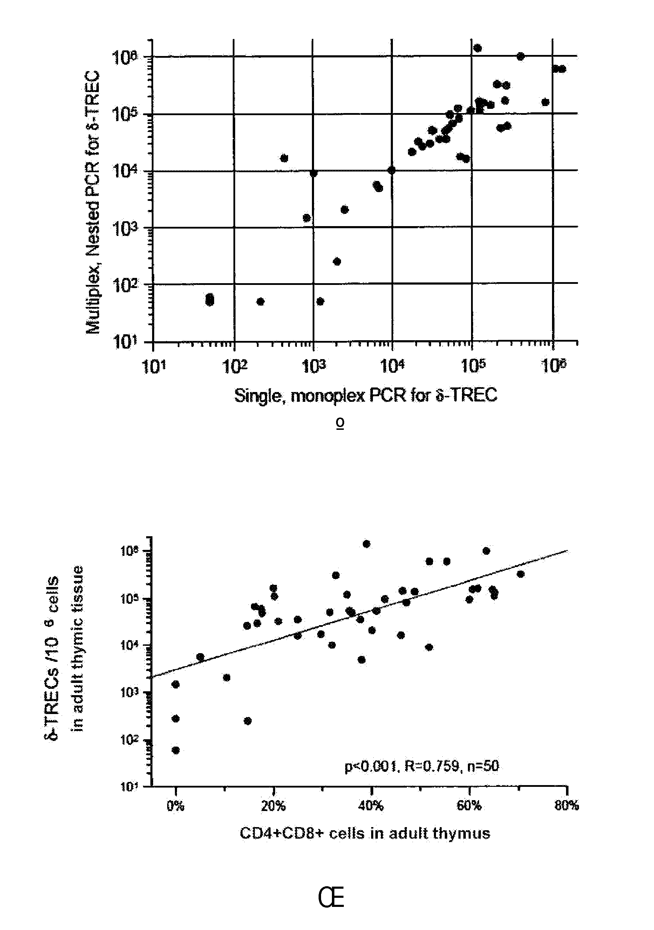

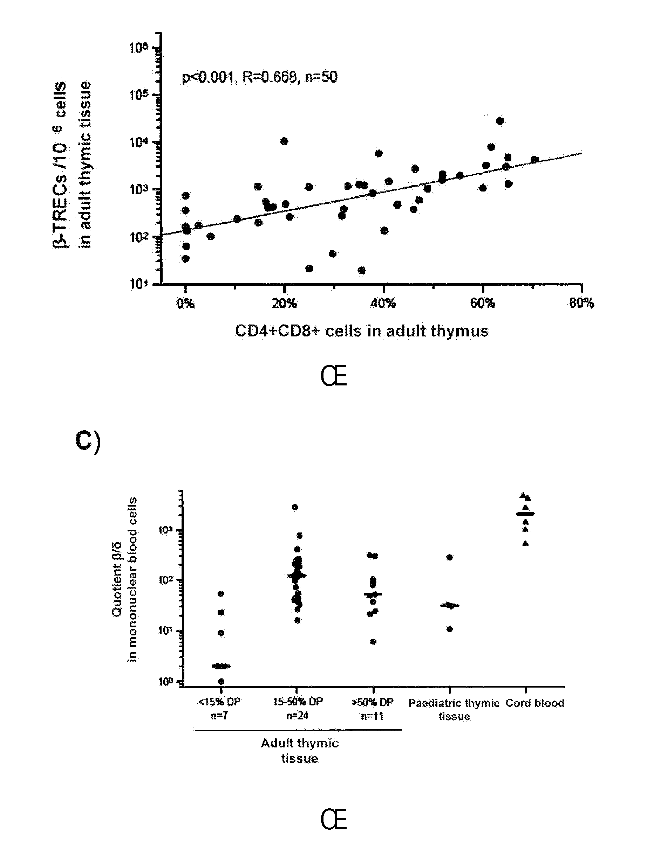

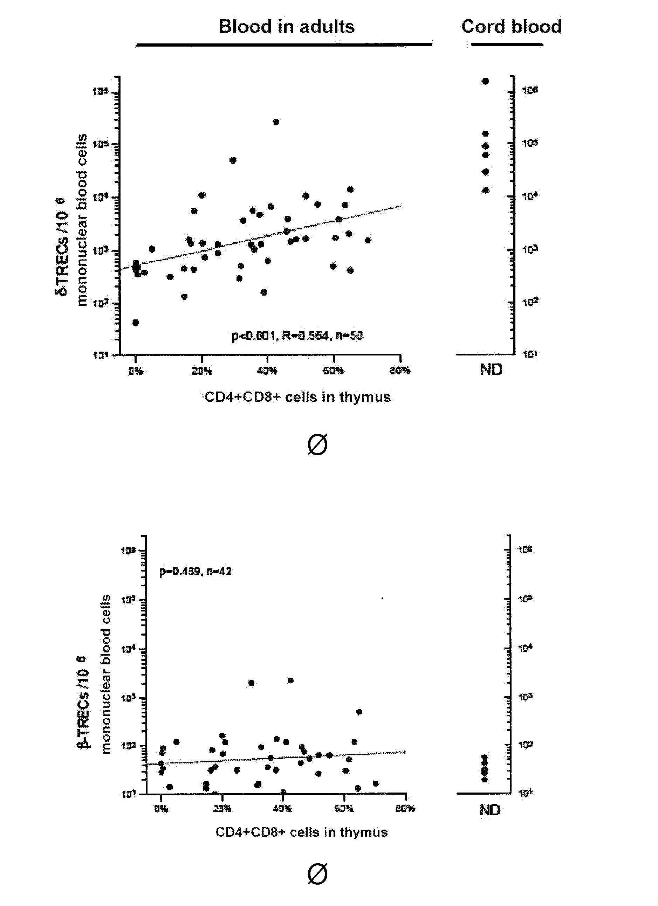

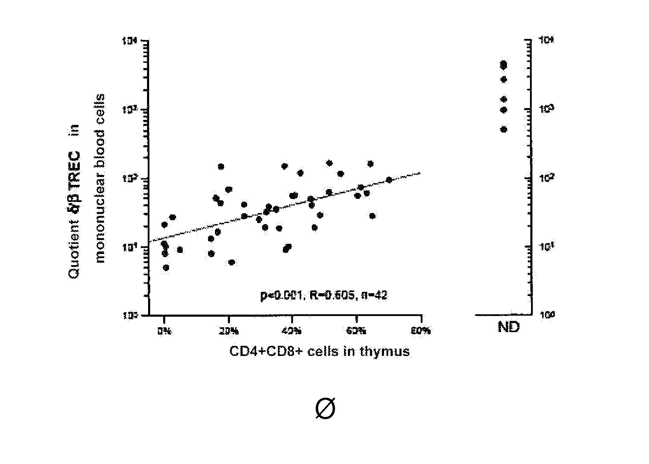

**Figure 1****:** Design of the multiplex PCR for the delta/beta TREC quotient. Schematic diagram of the PCR for the simultaneous amplification of delta and beta TREC. The pre-amplification PCR of step 1 (Figure 1 A) and 2 (Figure 1 B) are done separately, using a mixture of a single antisense primer and six sense primers, each one specific for a TCR-β reordering of Dβ1-Jβ1.1 to Dβ1-Jβ1.6, for step 1, and two external primers for delta TREC (step 2). Later, 1:20 dilutions of each first round of PCR (in triplicate) are re-amplified using real time PCR (Figure 1 C).
**Figure 2**: Simultaneous detection of the delta TREC signal (square, F2 channel for Red640 readings) and the integrated signal of six possible beta TREC (circles, F3 channel for Red705 readings). The amplifications of a representative sample are shown in triplicate.
**Figure 3**: Absence of saturation in the 20-cyle pre-amplification reaction compared to 16-cycle pre-amplification. Figure 3A shows the accumulated threshold cycle of both PCRs for the amplification of the cloned sequences of beta TREC after 20 (circles) or 16 cycles (square). The same for figure 3B (amplification of delta TREC sequences).
**Figure 4**: Interassay variability of the technique. The threshold cycle measured in the F2 and F3 fluorescence channels (hybridization probes of delta and beta TREC, respectively) for a same reaction tube is represented for each point of the straight-line measured in independent experiments. The dispersion is small for the most concentrated straight-line points.
**Figure 5**: Comparison of the delta TREC determination in thymic samples using the previously described technique of direct real time PCR, compared with the multiplex nested PCR for determining the delta/beta quotient. The paired values show identical measures with both techniques.
**Figure 6**: A) Correlation between the percentage of DP cells and the concentration of delta TREC per million thymic tissue cells. B) Correlation between the percentage of DP cells and the concentration of beta TREC per million thymic tissue cells. C) The δ/β TREC quotient measured in thymic samples stops working due to the presence of immature cells The quotients obtained in mature thymic tissue were grouped in three blocks in accordance with the percentage of double positive cells (DP) and they were compared with paediatric thymic tissue or with peripheral blood mononuclear cells (PBMC) obtained from the umbilical cord. The homogenous population of mature T-lymphocytes present in the umbilical cord have an average quotient of 1000, whilst the quotient in total thymic tissue is less, in particular in the most active thymuses.
**Figure 9**: Correlation between thymic function measured as percentage of double positive thymocytes (DP, CD4⁺CD8⁺) and delta/beta TREC quotient in peripheral blood mononuclear cells (PBMC) of adult individuals. A) Correlation between delta TREC content in PBMC and percentage of DP cells in the thymus; B) Representation of beta TREC content in PBMC compared with the percentage of double positive cells showing the absence of correlation; C) Correlation between the delta and beta TREC quotient in PBMC and percentage of DP cells in the thymus.

### DETAILED DESCRIPTION OF THE INVENTION

The general objective of the present invention is to develop a simplified method for determining, in biological samples, the quotient between the delta-type TREC (δRec-ΦJα) and a set of beta-type TREC (specifically six possible recombinations between segment Dβ1 and segments Jβ1 to Jβ6). In the state of the art (Dion et al., previously cited) for each individual TREC that participates in calculation of the quotient, a first multiplex PCR reaction is needed which pre-amplifies said TREC and a fragment of the CD3 gene as reference, followed by two real time PCR reactions, which finally calculate the concentration of said TREC and the fragment of the CD3 gene, separately. After normalization of the measurements using CD3, the individual values obtained for each beta TREC of the chosen set are added together. Depending on the number of beta-type TREC that one wants to include in the calculation (between six and ten) thus between seven and eleven conventional PCR and between fourteen and twenty-two real time PCR are needed.

The simplification obtained in the present invention fundamentally relates to a reduction in the number of PCR reactions and primers necessary to effectively measure said parameters. For this, the multiplex PCR approach is used differently, which makes it possible to reduce the number of PCR to be performed on two conventional and one real time PCR, if it uses, for example, a set of six beta-type TREC, resulting in a large sample economy. In its general design a multiplex PCR pre-amplification is performed of a set of beta TREC, in parallel with another pre-amplification of the single delta TREC, followed by final multiplex amplification of the set of beta TREC and delta TREC, in a single reaction tube. Using specific sequence fluorescent tubes, the amplification of delta TREC and the set of beta TREC amplified are detected in real time, which makes it possible to calculate the quotient in a single tube, without the need to normalize by the quantity of genomic DNA present in the preparation of DNA.

For the embodiment of the multiplex PCR pre-amplification of the chosen set of beta TREC, a minimum set of primers was designed with the following characteristics: a single antisense primer was used specific for segment Db1, and therefore, common to the set of beta TREC, and a group of sense primers, each one of them specific for one of six segments Jb1.1 to Jb1.6 (Figure 1A). The set of sense primers specific for segments Jβ1.1 to Jβ1.6 was designed with the condition that their 3' end is an identical sequence of at least three nucleotides, ideally more. Said identical sequence for the whole set of primers must naturally be present in all Jβ1 segments, since in the present invention a single consensus sequence is not used, but, at least three, preferably six sequences, each one specific for a Jβl segment. In a preferred embodiment of the present invention, a set of primers was selected with the CACAG sequence (SEQ. ID. 07) at its 3' end. Additionally, each one of the sense primers specific for segments Jβ1.1 to Jβ1.6 was synthesized with a tail added at their 5' end which had the following characteristics: being an arbitrary sequence designed to be compatible with the other primers used in the different multiplex PCR. Additionally, it was verified that they were not complementary of any known sequence in the human genome. The presence of said extension at 5' provides the following benefits to the state of the art:
1) The sequence of this extension at 5' is incorporated in the amplified DNA fragments, for which reason the primers are already completely complementary to the amplified sequences in the successive phases of denaturing /alignment, which produces two desired effects: in first place they generate a sequence at 5' common to six amplification products of beta TREC, which makes it possible to design a second PCR which uses said sequence, together with another antisense primer, more internal than that used in the first reaction. In this way, with only two primers six beta TREC can be amplified, in an approach we can consider as "semi-nesting", as only one of the second primers are internal to the amplification product (Figure 1 C).
2) Any amplification product derived from the pairing by error of two sense primers equipped with said extension (either dimers of primers or amplification products by non-specific pairings in different regions of the genome) will have complementary ends, which has been described to generate secondary structures which reduce the efficacy of its subsequent amplification (Brownie J, Shawcross S, Theaker J, Whitcombe D, Ferrie R, Newton C1 Little S. The elimination of primer-dimer accumulation in PCR. Nucleic Acids Res. 1997 Aug 15;25(16):3235-41).

To define said primers the sequence of segments Db1 and Jb1.1 to Jb1.6 deposited in the Genbank with reference number U66061 has been used as reference. For the design of the specific primers for the delta TREC the sequence contained in the locus of the TCR delta deposited in the Genbank with reference number AE000661 has been used as reference.

The number of PCR performed in the first beta and delta PCR must be included in the log-linear region which characterizes any PCR, before the appearance of the plateau phase and which cancels the quantitative property of the PCR. Ideally from 16 to 20 cycles are performed.

The amplification products of both first PCR cycles are subjected to a second multiplex PCR reaction wherein the sequences which are simultaneously amplified correspond to the TREC and at least one of the beta TREC sequences of the chosen set. The sample of nucleic acids to be amplified in the second reaction corresponds to a mixture of the products of the 1^{st} beta multiplex and the 1^{st} delta multiplex in equal parts. Said sample may be additionally diluted in a variable factor, ideally 1/20. The primers used in the second multiplex reaction meet the following condition: one of them corresponds to the sequence included as extension at 5' of the set of primers of segments Jb1.1 to Jb1.6, in isolated manner.

The antisense primer is designed on the Db1 region, being more internal than the one previously used.

The accumulation of the amplification products is measured during the second PCR reaction in each cycle, by real time PCR technology. The FRET technology enables the detection of characteristic sequences of delta TREC or of a sequence common to all possible beta TREC. Through the use of thermocyclers in real time, such as the LightCycler, it is possible to distinguish, using readings at different wavelengths, the quantity produced of a determined DNA sequence throughout each cycle. The fluorescence in one channel collects the amplification in the reaction capillary of the sequence amplified for the delta TREC. The fluorescence in another channel simultaneously measures the fluorescence emitted associated to the common sequence of all beta TREC. This fluorescence intensity is proportional to the sum of all possible amplified beta TREC, without possibility of distinguishing between the different amplified beta TREC. The objective of the present invention is to determine the value of the sum of all beta TREC, without determining each value individually.

In each PCR reaction and in parallel to the test samples a serialized dilution of a straight-line pattern is amplified which contains known concentrations of different cloning vectors each one of them containing the product of the first amplification reaction of delta TREC or beta TREC individually amplified. The concentration in number of delta TREC or sum of beta TREC per microlitre of DNA preparation is obtained by comparison of the threshold amplification cycle in the second real time PCR of the test sample with a serialized dilution of a standard sample with known concentrations of the sequences amplified by the plasmids. Therefore, the volume of sample to be loaded in each reaction tube must be identical between the test samples and the standard samples. The concentrations will be obtained from the interpolation of the threshold cycles of each test sample with the log-linear adjustment of the threshold cycle of each point of the standard straight line. The determination of the threshold cycle, characteristic of each PCR reaction, is performed using a mathematical analysis algorithm of the amplification curves of each individual PCR reaction. Preferably, that known as "second derivative method", included in the analysis software packet of the LightCycler (Roche), will be used. It is, however, possible that this volume is different for the delta PCR and the beta multiplex of the first round, due to the lower frequency of beta TREC present in the test sample typically analysed. In a preferred embodiment, this load volume per reaction tube is 5µL for the delta TREC PCR and 10 µl for the beta TREC multiplex PCR.

To calculate the quotient between delta and beta TREC it suffices to know the relative concentration of delta and beta-type TREC in a cellular preparation, without the need to know the genomic DNA concentration present in said preparation. With the present invention it is only necessary to know the relative concentration of delta and beta type TREC, and it is not necessary to determine the concentration using absorbance methods at 260 nm or preferably by quantitative PCR of a reference gene such as CD3, the beta chain of haemoglobin or any other sequence whose number of copies per cellular genome is known at present.

As complement to the method described here, to calculate the different TREC per million cells, the absolute number of copies of delta or beta-type TREC per microlitre of DNA preparation shall be normalized by the genomic DNA concentration. This is determined by measuring the number of beta-globin gene copies, considering the existence of two copies of said gene per cell, and expressing the frequency of TREC as number of circles per million total cells.

### EMBODIMENT OF THE INVENTION

### Example 1: Amplification conditions of the multiplex PCR for determining the delta/beta TREC quotient:

In the present invention, the quantitative PCR technique is used to calculate the concentrations and the corresponding quotient between a delta-type TREC and a subset of six beta-type TREC, in appropriate biological samples. A preferred embodiment involves the following general steps, schematized in figure 1:
1) Pre-amplification from a cellular DNA preparation of the set of six beta-type TREC derived from the recombinations between the Dβ1 gene segment and the Jβ1.1 to Jβ1.6 gene segment, using a single multiplex PCR reaction, performed in a conventional thermocycler (Figure 1 A)
2) Pre-amplification from a cellular DNA preparation of delta-type TREC derived from recombination of the δRec and ψJα gene segments, also performed in a conventional thermocycler (Figure 1 B)
3) Re-amplification of aliquots of the PCR reactions of steps 1) and 2) in a single multiplex PCR reaction in a thermocycler with real time monitoring of the progression of the amplified derivatives of delta TREC and of the set of six beta TREC, using FRET hybridization probe technology (Figure 1 C).

The different steps are described in detail below:

For the pre-amplification of delta and beta-type TREC, one starts from a human cellular DNA preparation obtained using a commercial kit according to the manufacturer's indications, appropriate for the extraction of thymic tissue (NucleoSpin® Tissue Kit) or mononuclear blood cells (NucleoSpin® Blood Kit), both from Macherey-Nagel, Germany. The DNA preparation thus obtained usually has a concentration between 10 and 150 ng/µL and it is used directly for PCR reactions. The determination of the TREC concentrations in each test sample is determined in triplicate, using for each PCR reaction three 10 µL aliquots of DNA for the case of beta TREC and three 5µL aliquots for delta TREC. Both reactions are performed independently, with identical final reaction volume conditions (50 µL), the reaction mixture being formed by 2U of catalyzing enzyme (EuroClone thermostable DNA polymerase), reaction buffer supplied by the manufacturer at a concentration of 1x, dNTPs, and final CfeMg concentration of 3 mM, varying the set of primers used in each case. Thus, for the pre-amplification of the set of six beta TREC performed in step 1 six "sense" primers are used of sequences SEQ ID.100 to SEQ ID. 105 (one for each possible Jβ1 segment present in the beta TREC), at a final concentration of 80 nM each, and an "antisense" primer (SEQ.ID. 106, specific for the Dβ1 segment, present in six beta TREC) at a final concentration of 280 nM. For the pre-amplification of delta TREC performed in step 2, the primers are used with sequences SEQ ID 109 and SEQ ID 110, at a concentration of 100 nM each.

Both amplification reactions of steps 1 and 2 are performed independently in a "Biometra Tgradient" thermocycler, with the following amplification conditions: initial denaturing for 5 minutes at 95° C, followed by cyclical denaturing phases during 20 seconds at 95° C, hybridization for 45 seconds at 57° C and extension for 30 seconds at 72° C, followed by a final extension phase for 5 minutes at 72° C. The number of amplification cycles varies depending on the nature of the sample, 16 cycles for DNA preparations obtained from paediatric thymic tissue or 20 cycles for samples of mononuclear blood cells.

For the second amplification round (step 2) a common 1:20 dilution is made of the amplification products of the first PCR, adding 10 µL of the pre-amplification of beta TREC and 10 µL of the pre-amplification of delta TREC at a volume of 180 µL of water for PCR. The triplicates of the independent amplification reactions are combined by pairs so that the second amplification is peralso performed in triplicate from three independent 1:20 dilutions. The reaction conditions of step 3 consist of the amplification of an aliquot of 3 µL of said 1:20 solution in a final reaction volume of 20 µL. The reaction mixture uses the FastStart DNA MasterPLUS kit (Roche Molecular Biochemicals, Mannheim, Germany), including the primers SEQ ID 107, SEQ ID 108 at a concentration of 200 nM, the primers SEQ ID 111, SEQ ID 112 at a concentration of 100 nM, and the aforementioned hybridization probes SEQ ID 113, SEQ ID 114 (Franco et al. "T-cell repopulation and thymic volume in HIV-1-infected adult patients after highly active antiretroviral therapy"; Blood 2002 Vol. 99 3702-3706) and SEQ ID 115, SEQ ID 116 (Dion et al. "HIV infection rapidly induces and maintains a substantial suppression of thymocyte proliferation"; Immunity 2004 Vol. 21 757-768), at a final concentration of 100 nM and the acceptor fluorophores, type FRET Red640 and Red705, respectively (see Table 3). For the real time PCR a LightCycler® thermocycler was used, with an initial denaturing step of 10 minutes at 95°-C followed by 45 denaturing cycles (10 seconds at 95ºC), alignment (20 seconds at 57ºC) and extension (15 seconds at 726ºC). The fluorescence measurements are made at the end of the alignment phase.

Figure 2 shows the fluorescence measurements obtained for each amplification cycle corresponding to amplification of delta TREC (square, fluorescence emitted by Red605) and of beta TREC (circles, fluorescence emitted by Red740) in a representative sample.

### Example 2: Characterization of the multiplex PCR for determining the delta/beta TREC quotient:

To characterize the test properties, the DNA sequences characteristically amplified in the two pre-amplification reactions were cloned in pGEM-T Easy cloning vectors (Promega, Madison, USA) following the manufacturer's instructions. Clones were obtained corresponding to delta TREC and beta TREC Dβ1 :Jβ1.1, Dβ1:Jβ1.2, Dβ1: β1.3 and Dβ1 :Jβ1.4.

Once preparations of known concentration are obtained of each individual plasmid, a mixture base dilution was performed of said plasmids characterized in that they contain 4747 copies/µL of the cloning vector which contains the amplified sequence of delta TREC and 189 copies/µL of a mixture in equal parts of the cloning vectors containing beta TREC Jβ1.1 to Jβ1.4. Said concentrations correspond to a characteristic delta TREC /Σ betas quotient for the straight-line pattern of 25, irrespective of the dilution used.

Various assay parameters were analysed using said straight-line pattern, in 30 independent reactions. For each assay, a 1:10 serialized dilution was made of the aforementioned standard curve, and 10 and 5 µL aliquots were amplified according to the conditions described in steps 1 and 2 of example 1, respectively. The different dilutions of the straight-line provided, therefore, an average of 23735 to 23.7 delta-type amplifiable sequences and 1890 to 1.9 beta-type amplifiable sequences per tube, respectively.

The sensitivity analysis of the assay described in example 1 were capable of amplifying all the dilutions containing delta-type sequences, as well as 97% of the reactions containing an average of 19 beta-type amplifiable sequences per tube, and 75% of the reactions containing 1.9 sequences per tube. These frequencies are in accordance with the expected tube distribution that lack amplifiable sequences following a normal distribution of an average of 1.9, which shows that the assay has sufficient sensitivity to amplify from only one copy of DNA mould specific per reaction tube.

To evaluate if the pre-amplification reactions may have missed the linear amplification phase and entered in the plateau phase, the mean of the values calculated in the real time PCR was calculated for each point of the standard curve, after 16 or 20 pre-amplification cycles. The number of previously performed cycles are added to the threshold cycle calculated in the second PCR. The result of said sum (mean ± standard deviation) is shown in figure 3A for the case of amplification of beta-type TREC sequences, and in figure 3B for the case of amplification of delta-type TREC sequences, after 16 (square) or 20 (circles) pre-amplification cycles, showing the linearity of the technique until 20 cycles of the first PCR.

To analyse the reproducibility of the assay between experiments, the pairs of threshold cycles calculated in the second round of PCR for the fluorescence signal of channel F2 (delta TREC) and channel F3 (beta TREC) were represented in figure 4. The symbols represent the points of the straight-line pattern without diluting (inverted triangles) or serially diluted in a factor of 10 (triangles, circles and squares respectively).

### Example 3: Maintenance of the reordering of delta TCR in the thymus of advanced age despite the lack of double positive cells (DP):

The mechanisms which lead to age-associated thymic involution are not known. The thymus lacks a population of cells which are capable of self-renewing themselves, for which reason they need haematopoietic precursors of the bone marrow to maintain thymopoiesis. Thus, it is generally accepted that involution of the thymus is due to a reduction in the quantity of progenitors that colonize the thymus. However, a reduction has recently been shown of age-related intrathymic proliferation as cause of thymus involution. To evaluate if the delta/beta TREC quotient really measures thymic function, the relation was analysed between the percentage of double positive thymocytes and the delta/beta TREC quotient of this thymic tissue samples. Some paediatric thymuses were analysed as controls.

The ages of the 50 patients analysed were included between 36 and 81 years of age (median 60 years of age, interquartile ranges 59-73 years of age). 60% were men. As expected, the degree of residual thymopoiesis was variable, ranging from 0 to 71 % of double positive (median 32%, interquartile range 15-50%). A direct and significant correlation was found between the percentage of double positive cells and the delta TREC values per total million thymic tissue cells (p<0.001, r=0.759, n=50, Figure 6A). Furthermore, the beta TREC content per million tissue cells also correlates with the percentage of DP (p<0.001, r=0.668, n=50, Figure 6B).

Figure 6C shows the delta/beta TREC quotient in adult thymus samples grouped as percentage of double positives as very reduced (0- 15%), moderate (15-50%) or almost normal (>50%). These quotients were compared with samples of paediatric thymus and cord mononuclear blood cells. Mann-Whitney's U-test showed significant differences between all groups with paediatric thymus, except for the almost normal double positives group. As the single positive cells are the subpopulation of the inthrathymic proliferation evaluated directly by the delta/beta TREC quotient, greater quotients were expected in the paediatric thymuses and in the samples with more than 50% DP. However, figure 6C shows unexpectedly low quotients for these groups compared with the samples with a percentage of DP between 15 and 50% or with the cord blood cells (p<0.001). It is probably due to the presence in the thymus of immature positive cells for beta TREC and negative for delta TREC, altering the quotient of the entire thymopoietic series. Thus, to adequately measure the quotient in thymocytes with a fixed measurement it is necessary to previously isolate the single positive CD4 and CD8 cells.

Finally, 9 thymuses were detected of the 50 analysed without double positive thymocytes. The cells isolated from these thymuses had cytometry profiles compatible with lymphoid cells, including CD3+CD45RA cells- single positive, suggesting a certain recirculation of mature lymphocytes. Two samples were negative both for beta TREC and for delta TREC, however, the seven remaining ones were positive for beta TREC but negative for delta TREC or with a delta/beta TREC quotient close to one. This suggests that even in the total absence of DP a certain number of stem cells still colonize the thymus and start the reordering of the TCR-β. The lack of delta TREC and DP points to a drastic reduction in intrathymic proliferation associated to an effective reordering of the TCR beta chain, the assay described here being capable of detecting these residual reorderings.

### Example 4: Comparison of the measurement of delta TREC using multiplex nested PCR with a direct delta TREC assay:

In order to compare the measurement of the delta TREC obtained according to the assay described in example 1 with the measurements obtained by a real time PCR assay for direct quantification of the aforementioned delta TREC (Franco et al. "T-cell repopulation and Thymic volume in HIV-1-infected adult patients after highly active antiretroviral therapy"; Blood 2002 Vol. 15 3702-3706), the thymic tissue samples of example 3 were measured by both assayed.

Briefly, a direct amplification of delta TREC was additionally performed using the primers SEQ ID 117 and SEQ ID 118 (see Table 3), using LightCycler® FastStart DNA MasterPLUS, with an initial denaturing step at 95ºC for 10 minutes followed by 40 denaturing cycles (10 seconds at 95ºC), alignment (15 seconds at 50ºC) and extension (20 seconds at 72ºC). The fluorescence measurements are performed at the end of the alignment phase using the hybridization probes SEQ ID 113 and SEQ ID 114 (see Table 3).

To express the TREC content as number of cycles per million cells, primers SEQ ID 119 and SEQ ID 120 were used (see Table 3) to quantify the human beta-globin gene according to the aforementioned protocol (Franco et al. "T-cell repopulation and Thymic volume in HIV-1-infected adult patients after highly active antiretroviral therapy"; Blood 2002 Vol. 15 3702-3706). It was considered that each cell carries two copies of said sequence. The PCR reaction was performed in duplicate using LightCycler® FastStart DNA MasterPLUS SYBR Gren I. After an initial denaturing cycle at 95ºC for 10 minutes 40 denaturing cycles were performed (10 seconds at 95ºC), alignment (10 seconds at 66ºC) and extension (15 seconds at 72ºC). The fluorescence of the SYBR Green I is measured at the end of the extension phase.

Figure 5 shows the correlation between the measurements obtained with both techniques. The linear regression adjusted to the logarithmically expressed measurements showed a gradient of 1.005 (p<0.001, r=0.916, n=47), indicating that the delta TREC is measured in similar fashion with both assays.

### Example 5: The delta/beta TREC quotient in peripheral blood directly correlates to residual thymic function

The relation was analysed between the thymic function measured directly from thymic tissue and the TREC in peripheral blood mononuclear cells (PBMCs).

Figure 7A shows a direct correlation between the delta TREC values in the blood and the percentage of double positive cells in the thymus (p<0.001, r=0.564, n=50). The quantity of beta TREC per million PBMCs (Figure 7B) does not correlate with the percentage of DP (p=0.288). The beta TREC content in adult PBMCs is similar to that of cord blood (145±56 and 45±6 beta TREC per million PBMCs, respectively). Eight samples were negative for the beta TREC PCR, for which reason it was not possible to calculate the delta/beta TREC quotient. The delta/beta TREC quotient (Figure 7C) in other peripheral blood samples showed a direct correlation with the percentage of DP cells in thymic tissue samples (p<0.001, r=0.605, n=42), showing their use in the measurement of the thymic function. These results agree with those obtained from mathematical models (Van den Dool et al. "The effects of age, thymectomy, and HIV infection on alpha and beta TCR excision circles in naive T cells"; J Immunol 2006 Vol. 177 4391-4401). The fraction of recent thymic emigrants (RTEs) which have beta TREC strongly depend on intrathymic proliferation that the cord blood cells must have lower beta TREC levels than individuals of age. In a thymic involution model that affects intrathymic proliferation, the total beta TREC values exported to the periphery must exclusively depend on how many haematopoietic stem cells started to reorganize their TCR-β locus. The quantity of intrathymic proliferation has an effect on the proportion of RTEs that conserve a beta TREC, but the absolute number of beta TREC exported must be constant. Thus, the frequency of beta TREC in PBMCs must be maintained constant irrespective of the quantity of intrathymic proliferation, as suggested in the aforementioned mathematical model.

Intrathymic proliferation is a parameter fixed during T-cell differentiation and is not later altered by the additional proliferation of the mature cells. For this reason, it is not necessary to isolate the subpopulations of T-cells to eliminate the memory or activated T-cells. However, intrathymic proliferation can be considered an "instantaneous" parameter: it is a property of a cohort of RTEs that have just abandoned the thymus. If the thymus was capable of modulating its activity in stimulating drugs or other stimulants in a short period of time, the delta/beta TREC quotient could be indicative of these changes.

It would be more interesting to isolate the different subpopulations of the peripheral blood mononuclear cells before determining the delta/beta TREC quotient. The population of T-cells most rich in RTEs should be isolated, isolating the CD27+ virgin T-cells or the CD45RA+CD31+ subpopulation, which will provide more exact details. Thus, the multiplex PCR will need a much greater quantity of DNA.

The quotient in PBMCs also shows a direct correlation with the percentage of CD45RA+CD27+ virgin cells both in the CD4+ (p<0.001, r=0.531) and in the CD8+ (p<0.001, r=0.511) subpopulation (data not shown). No correlation was found with age (p=0.770, n=42). Significant differences were showed by sex, with women showing greater ratios (66.5±12 vs. 37.3±7, p=0.015, Mann-Witney's U test).

**Table 1**

| **Sequences of 5 nucleotides common to all primers at their 3' end** | | | |
|---|---|---|---|
| | | | |
| SEQ. ID. 1 | ACCAG | SEQ. ID. 23 | GCAGA |
| SEQ. ID. 2 | AGACT | SEQ. ID. 24 | GCTCA |
| SEQ. ID. 3 | AGAGT | SEQ. ID. 25 | GGCAG |
| SEQ. ID. 4 | AGGGA | SEQ. ID. 26 | GGGAG |
| SEQ. ID. 5 | AGTGG | SEQ. ID. 27 | GGGGT |
| SEQ. ID. 6 | ATTTT | SEQ. ID. 28 | GGGTA |
| SEQ. ID. 7 | CACAG | SEQ. ID. 29 | GGGTC |
| SEQ. ID. 8 | CACTG | SEQ. ID. 30 | GGTGG |
| SEQ. ID. 9 | CAGAA | SEQ. ID. 31 | GTAAG |
| SEQ. ID. 10 | CAGCT | SEQ. ID. 32 | GTCTT |
| SEQ. ID.11 | CAGGG | SEQ. ID. 33 | GTTCT |
| SEQ. ID. 12 | CCAGA | SEQ. ID. 34 | TCAGG |
| SEQ. ID. 13 | CCAGG | SEQ. ID. 35 | TCCCT |
| SEQ. ID. 14 | CCTCT | SEQ. ID. 36 | TCCTC |
| SEQ. ID. 15 | CTCCT | SEQ. ID. 37 | TCTCT |
| SEQ. ID. 16 | CTCTC | SEQ. ID. 38 | TCTTT |
| SEQ. ID. 17 | CTCTG | SEQ. ID. 39 | TGGGG |
| SEQ. ID. 18 | CTGTC | SEQ. ID. 40 | TGGGT |
| SEQ. ID. 19 | CTGTG | SEQ. ID. 41 | TTCTT |
| SEQ. ID. 20 | GAAGG | SEQ. ID. 42 | TTTGG |
| SEQ. ID. 21 | GACTC | SEQ. ID. 43 | TTTTG |
| SEQ. ID. 22 | GAGAA | SEQ. ID. 44 | TTTTT |

**Table 2**

| **Sequences of 4 nucleotides common to all primers at their 3' end** | | | |
|---|---|---|---|
| | | | |
| SEQ. ID. 45 | AAAG | SEQ. ID. 73 | GGAC |
| SEQ. ID. 46 | AAGA | SEQ. ID. 74 | GGCA |
| SEQ. ID. 47 | AAGG | SEQ. ID. 75 | GGGA |
| SEQ. ID. 48 | ACAG | SEQ. ID. 76 | GGGT |
| SEQ. ID. 49 | ACTC | SEQ. ID. 77 | GGTA |
| SEQ. ID. 50 | ACTG | SEQ. ID. 78 | GGTC |
| SEQ. ID. 51 | AGAC | SEQ. ID. 79 | GGTT |
| SEQ. ID. 52 | AGAG | SEQ. ID. 80 | GTAA |
| SEQ. ID. 53 | AGCT | SEQ. ID. 81 | GTCA |
| SEQ. ID. 54 | AGGG | SEQ. ID. 82 | GTGG |
| SEQ. ID. 55 | AGGT | SEQ. ID. 83 | GTGT |
| SEQ. ID. 56 | ATGT | SEQ. ID. 84 | GTTC |
| SEQ. ID. 57 | CACA | SEQ. ID. 85 | TAAG |
| SEQ. ID. 58 | CACT | SEQ. ID. 86 | TATG |
| SEQ. ID. 59 | CAGA | SEQ. ID. 87 | TCAG |
| SEQ. ID. 60 | CAGG | SEQ. ID. 88 | TCCA |
| SEQ. ID. 61 | CCAG | SEQ. ID. 89 | TCTT |
| SEQ. ID. 62 | CCCT | SEQ. ID. 90 | TGAA |
| SEQ. ID. 63 | CCTT | SEQ. ID. 91 | TGGA |
| SEQ. ID. 64 | CTCA | SEQ. ID. 92 | TGGC |
| SEQ. ID. 65 | CTGA | SEQ. ID. 93 | TGGG |
| SEQ. ID. 66 | CTTT | SEQ. ID. 94 | TGTC |
| SEQ. ID. 67 | GAAA | SEQ. ID. 95 | TGTG |
| SEQ. ID. 68 | GAAG | SEQ. ID. 96 | TTCT |
| SEQ. ID. 69 | GACT | SEQ. ID. 97 | TTGG |
| SEQ. ID. 70 | GAGT | SEQ. ID. 98 | TTTG |
| SEQ. ID. 71 | GCAG | SEQ. ID. 99 | TTTT |
| SEQ. ID. 72 | GCTT | | |

**Table 3**

| **Sequences from the prior art used in the embodiment of the invention** | |
|---|---|
| | |
| SEQ ID 113 | AGG GAT GTG GCA TCA CCT TTG TTG ACA |
| SEQ ID 114 | GGC ACC CCT CTG TTC CCC ACA GGA |
| SEQ ID 115 | CTG GGA GTT GGG ACC GCC AGA GAG GT |
| SEQ ID 116 | TTT GTA AAG GTT TCC CGT AGA GTT GAA TCA TTG TG |
| SEQ ID 117 | AGG CTG ATC TTG TCT GAC ATT TGC TCC G |
| SEQ ID 118 | AAA GAG GGC AGC CCT CTC CAA GGC |
| SEQ ID 119 | CAA CTT CAT CCA CGT TCA CC |
| SEQ ID 120 | GAA GAG CCA AGG ACA GGT AC |

## Claims

1. Set of primers for determining the functionality of the human thymus by calculating the quotient between delta and beta TREC content, **characterized in that** said set includes at least three primers each one of which have a sequence of nucleotides which is complementary with a region between nucleotides 187957 and 190719 of the sequence deposited in the Genbank database with access number U66061, as well as a sequence of nucleotides at their 5' end which is substantially non-complementary with any known region of the human genome.

2. Set of primers for determining the functionality of the human thymus according to claim 1, **characterized in that** the primers of said set have at their 3' end a same common sequence to all of them, which is either identical, or complementary, to one of sequences SEQ. ID. 01 to SEQ. ID. 99.

3. Set of primers for determining the functionality of the human thymus according to claims 1 and 2, **characterized in that** said primers have at their 3' end sequence SEQ. ID. 07.

4. Set of primers for determining the functionality of the human thymus according to claims 1 to 3, **characterized in that** said set additionally includes one of sequences SEQ. ID. 100 to SEQ. ID. 112

5. Set of primers for determining the functionality of the human thymus according to claim 4, **characterized in that** said set of primers is formed by:
a) six primers which contain sequences SEQ. ID. 100 to SEQ. ID. 105.
b) two primers which contain sequences SEQ. ID. 106 and SEQ. ID. 107.
c) a primer which contains sequence SEQ. ID. 108.
d) four primers which contain sequences SEQ. ID. 109, SEQ. ID. 110, SEQ. ID.111 and SEQ. ID. 112.

6. Method for determining the functionality of the human thymus which includes simultaneous amplification stages of more than one beta or delta-type TREC by polymerase chain reaction (multiplex PCR), **characterized in that** said method comprises:
a) performing an amplification reaction from a cellular DNA preparation using a specific primer of a sequence common to the set of TREC of the beta family to be amplified, and a set of specific primers for each individual TREC of the beta family to be amplified, which have at their 5' end an identical sequence for all of them and which is substantially non-complementary with any known region of the human genome;
b) performing in parallel, but independently, an amplification reaction from a cellular DNA preparation using a pair of specific primers of the sequence of specific bases of a delta-type TREC;
c) performing a single amplification reaction from a mixture of aliquots of said previous amplification reactions, wherein at least one primer is used which comprises the sequence present at the 5' end of the set of primers used in stage a).

7. Method for determining the functionality of the human thymus according to claim 6, **characterized in that** the aliquots of the amplification products of the first amplification reactions are used directly or diluted at least in the proportion 1:2, preferably 1:20 .

8. Method for determining the functionality of the human thymus according to claims 6 and 7, **characterized in that** the detection of the products of the polymerase chain reaction of the set of delta and beta TREC are performed in real time using a hybridization probe common for all the beta TREC and a hybridization probe specific for delta TREC, the signals of both probes being distinguishable.

9. Method for determining the functionality of the human thymus according to claims 6-8, **characterized in that** the detection of the polymerase chain reaction products of the set of delta and beta TREC are performed using fluorescence probes.

10. Method for determining the functionality of the human thymus according to claims 6 to 9, **characterized in that** the primers used correspond to sequences SEQ. ID. 100 to SEQ. ID. 112, being performed according to the following stages:
a) a first amplification round of six β-TREC in a single multiplex PCR reaction using six sense primers (SEQ. ID. 100 to SEQ. ID. 105) specific for each β-TREC and a single antisense primer (SEQ. ID. 106) specific for a constant region of six β-TREC and, in parallel, a first amplification round of δ-TREC using PCR using the primers which contain sequences SEQ. ID. 109 and SEQ. ID. 1 10, all from cellular DNA samples, particularly of blood or thymus cells;
b) a second amplification round set of aliquot dilutions of at least 1:2, preferably 1:20, of each one of the amplifications performed in the previous step using a single multiplex PCR which uses the primers which contain sequences SEQ. ID. 107 and SEQ. ID. 108 for the β-TREC amplified in the previous step and the primers which contain sequences SEQ. ID. 111 and SEQ. ID. 112 for the δ-TREC amplified in the previous step;
c) calculation of the quotient between the quantity of δ-TREC and the total quantity of β-TREC.

11. Method for determining the functionality of the human thymus according to claims 6 to 10, **characterized in that** a serialized dilution of a straight-line pattern is amplified, in each PCR reaction and in parallel to the test samples, which contains known concentrations of the products of the first δ-TREC or β-TREC amplification reaction, so that it can be compared with the values obtained with the test samples in the threshold amplification cycle of the second real time PCR, obtaining the concentrations from the interpolation of the threshold cycles of each test sample with the logarithmic-linear adjustment of the threshold cycle of the straight-line pattern.

12. Method for determining the functionality of the human thymus according to claims 6 to 11, **characterized in that** the absolute number of copies of δ-TREC or β-TREC per microlitre of cellular DNA preparation is normalized from the number of copies of the beta-globin gene, with two copies of said gene per cell, expressing the frequency of said TREC as number of circles per million total cells.

13. Set of tools for determining the functionality of the human thymus using a method according to claims 6 to 12, **characterized in that** it includes:
a) set of primers which contain sequences SEQ. ID. 100 to SEQ. ID. 112
b) hybridization probes that allow differentiation between β-TREC and δ-TREC;
c) cloning vectors with known concentrations of β-TREC and δ-TREC that make it possible to construct a straight-line pattern.

14. Set of tools for determining the functionality of the human thymus according to claim 13, **characterized in that** the hybridization probes that allow differentiation between β-TREC and δ-TREC are fluorescence probes.


SEQUENCE LISTING

<110> Fundación Reina Mercedes para la Investigación Sanitaria
Fundación para la Investigación y Prevención del SIDA en
España

<120> PRIMERS, METHOD AND SET OF TOOLS FOR DETERMINING THE FUNCTIONALITY
OF THE HUMAN THYMUS

<130> 1985.30

<140> P200702224
<141> 2007-08-02

<160> 21

<170> PatentIn version 3.5

<210> 100
<211> 38
<212> DNA
<213> Artificial Sequence

<220>
<223> Primer PCR

<400> 100
cggccgtagc gacgtaccct ttcgatggac cctcacag 38

<210> 101
<211> 39
<212> DNA
<213> Artificial Sequence

<220>
<223> Primer PCR

<400> 101
cggccgtagc gacgtaccga caaggcacca gactcacag 39

<210> 102
<211> 39
<212> DNA
<213> Artificial Sequence

<220>
<223> Primer PCR

<400> 102
cggccgtagc gacgtaccaa gctctggaag ggaacacag 39

<210> 103
<211> 39
<212> DNA
<213> Artificial Sequence

<220>
<223> Primer PCR

<400> 103
cggccgtagc gacgtacccc gtttctctcc ctcacacag 39

<210> 104
<211> 39

<212>  DNA
<213>  Artificial Sequence

<220>
<223>  Primer PCR

<400>  104
cggccgtagc gacgtaccgg gcagaaactg agaacacag                              39


<210>  105
<211>  39
<212>  DNA
<213>  Artificial Sequence

<220>
<223>  Primer PCR

<400>  105
cggccgtagc gacgtaccct tgcgccttat gctgcacag                              39


<210>  106
<211>  22
<212>  DNA
<213>  Homo sapiens

<400>  106
tgaaccaaat tgcattaaga cc                                                22


<210>  107
<211>  21
<212>  DNA
<213>  Homo sapiens

<400>  107
cccaggagga aagaagagga c                                                 21


<210>  108
<211>  18
<212>  DNA
<213>  Artificial Sequence

<220>
<223>  Primer PCR

<400>  108
cggccgtagc gacgtacc                                                     18


<210>  109
<211>  20
<212>  DNA
<213>  Homo sapiens

<400>  109
agaaggctct gtctagtgtg                                                   20


<210>  110
<211>  18
<212>  DNA
<213>  Homo sapiens

<400>  110
tctgacattt gctccgtg                                                     18

<210> 111
<211> 22
<212> DNA
<213> Homo sapiens

<400> 111
aggctctgtc tagtgtgata ac 22

<210> 112
<211> 18
<212> DNA
<213> Homo sapiens

<400> 112
tgacatggag ggctgaac 18

<210> 113
<211> 24
<212> DNA
<213> Homo sapiens

<400> 113
agggatgtgg catcaccttt gaca 24

<210> 114
<211> 24
<212> DNA
<213> Homo sapiens

<400> 114
ggcacccctc tgttccccac agga 24

<210> 115
<211> 26
<212> DNA
<213> Homo sapiens

<400> 115
ctgggagttg ggaccgccag agaggt 26

<210> 116
<211> 35
<212> DNA
<213> Homo sapiens

<400> 116
tttgtaaagg tttcccgtag agttgaatca ttgtg 35

<210> 117
<211> 28
<212> DNA
<213> Homo sapiens

<400> 117
aggctgatct tgtctgacat ttgctccg 28

<210> 118
<211> 24
<212> DNA
<213> Homo sapiens

<400>  118
aaagagggca gccctctcca aggc                                              24


<210>  119
<211>  20
<212>  DNA
<213>  Homo sapiens

<400>  119
caacttcatc cacgttcacc                                                   20


<210>  120
<211>  20
<212>  DNA
<213>  Homo sapiens

<400>  120
gaagagccaa ggacaggtac                                                   20